(19) Europäisches Patentamt
European Patent Office
Office européen des brevets

(11) Publication number: 0 331 803
A2

(12) EUROPEAN PATENT APPLICATION

(21) Application number: 88120133.9

(22) Date of filing: 02.12.88

(51) Int. Cl.[4]: A61K 45/06 , A61K 37/64 , //(A61K37/64,31:55,31:44)

(30) Priority: 07.03.88 US 164689

(43) Date of publication of application: 13.09.89 Bulletin 89/37

(84) Designated Contracting States: AT BE CH DE ES FR GB GR IT LI LU NL SE

(71) Applicant: E.R. Squibb & Sons, Inc.
Lawrenceville-Princeton Road
Princeton, N.J. 08540(US)

(72) Inventor: Sudilovsky, Abraham
712 Winchester Avenue
Lawrenceville New Jersey(US)

(74) Representative: Perani, Aurelio et al
c/o JACOBACCI-CASETTA & PERANI S.p.A 7,
Via Visconti di Modrone
I-20122 Milano(IT)

(54) **Method for inhibiting onset of or treating migraine headaches employing an ace inhibitor.**

(57) A method is provided for inhibiting onset of or treating migraine headache by administering an ACE inhibitor, such as captopril, alone or in combination with a calcium channel blocker such as diltiazem or nifedipine, over a prolonged period of treatment.

EP 0 331 803 A2

# METHOD FOR INHIBITING ONSET OF OR TREATING MIGRAINE HEADACHES EMPLOYING AN ACE INHIBITOR

The present invention relates to a method for inhibiting onset of or treating migraine headaches by administering an ACE inhibitor, such as captopril, zofenopril, fosinopril or enalapril, alone or in combination with a calcium channel blocker, such as diltiazem or nifedipine.

In accordance with the present invention, a method is provided for inhibiting onset of or treating migraine headaches wherein a therapeutically effective amount of an angiotensin converting enzyme inhibitor alone or in combination with a calcium channel blocker is systemically, such as orally or parenterally, administered over a prolonged period, whereby frequency and intensity of migraine headaches are significantly reduced.

Where a combination of ACE inhibitor and calcium channel blocker are to be used, the ACE inhibitor will be employed in a weight ratio to the calcium channel blocker of within the range of from about 0.1:1 to about 10:1 and preferably from about 0.4:2 to about 2.5:1.

The angiotensin converting enzyme inhibitor which may be employed herein includes substituted proline derivatives, such as any of those disclosed in U.S. Patent No. 4,105,776 to Ondetti et al, with captopril, that is, 1-[(2S)-3-mercapto-2-methylpropionyl]-L-proline, being preferred, carboxyalkyl dipeptide derivatives, such as any of those disclosed in U.S. Patent No. 4,374,829 with N-(1-ethoxycarbonyl-3-phenylpropyl)-L-alanyl-L-proline, that is, enalapril, being preferred.

Other examples of angiotensin converting enzyme inhibitors suitable for use herein include any of the phosphonate substituted amino or imino acids or salts disclosed in U.S. Patent No. 4,452,790 with (S)-1-[6-amino-2-[[hydroxy-(4-phenylbutyl)phosphinyl]oxy]-1-oxohexyl]-L-proline being preferred, phosphinylalkanoyl prolines disclosed in U. S. Patent No. 4,168,267 with fosinopril being preferred, mercaptoacyl derivatives of substituted prolines, disclosed in U.S. Patent No. 4,316,906 with zofenopril being preferred, any of the phosphinylalkanoyl substituted prolines disclosed in U.S. Patent No. 4,337,201 and the phosphonamidates disclosed in U.S. Patent No. 4,432,971.

Other examples of ACE inhibitors that may be employed herein include Beecham's BRL 36,378 as disclosed in European patent Nos. 80822 and 60668; Chugai's MC-838 disclosed in CA. 102:72588v and Jap. J. Pharmacol. 40:373 (1986); Ciba-Geigy's CGS 14824 (3-([1-ethoxycarbonyl-3-phenyl-(1S)-propyl]-amino)-2,3,4,5-tetrahydro-2-oxo-1-(3S)-benzazepine-1 acetic acid HCl) disclosed in U.K. Patent No. 2103614 and CGS 16,617 (3(S)-[[(1S)-5-amino-1-carboxypentyl]amino]-2,3,4,5-tetrahydro-2-oxo-1H-1-benzazepine-1-ethanoic acid) disclosed in U. S. Patent No. 4,473,575; cetapril (alacepril, Dainippon) disclosed in Eur. Therap. Res. 39:671 (1986); 40"543 (1986); ramipril (Hoechst) disclosed in Eur. Patent No. 79-022 and Curr. Ther. Res. 40:74 (1986); Ru 44570 (Hoechst) disclosed in Arzneimittelforschung 35:1254 (1985), cilazapril (Hoffman-LaRoche) disclosed in J. Cardiovasc. Pharmacol. 9:39 (1987); $R_o$ 31-2201 (Hoffman-LaRoche) disclosed in FEBS Lett. 165:201 (1984); lisinopril (Merck) disclosed in Curr. Therap. Res. 37:342 (1985) and Eur. patent appl. No. 12-401, indalapril (delapril) disclosed in U. S. Patent No. 4,385,051; rentapril (fentiapril, Santen) disclosed in Clin. Exp. Pharmacol. Physiol. 10:131 (1983); indolapril (Schering) disclosed in J. Cardiovasc. Pharmacol. 5:643, 655 (1983); spirapril (Schering) disclosed in Acta. Pharmacol. Toxicol. 59 (Supp. 5):173 (1986); perindopril (Servier) disclosed in Eur. J. Clin. Pharmacol. 31:519 (1987); quinapril (Warner-Lambert) disclosed in U.S. Patent No. 4,344,949 and CI 925 (Warner-Lambert) ([3S-[2[R(*)R(*)]]3R-(*)]-2-[2-[[1-(ethoxycarbonyl)-3-phenylpropyl]amino[-1-oxopropyl]-1,2,3,4-tetrahydro-6, 7-dimethoxy-3-isoquinolinecarboxylic acid HCl) disclosed in Pharmacologist 26:243, 266 (1984), WY-44221 (Wyeth) disclosed in J. Med. Chem. 26:394 (1983).

The calcium antagonist which will be used herein may be diltiazem which is disclosed in U. S. Patent No. 3,562,257 and which has the chemical name 3-(acetyloxy)-5-[2-(dimethylamino)ethyl-2,3-dihydro-2-(4-methoxyphenyl)-1,5-benzothiazepin-4(5H)-one and the structure

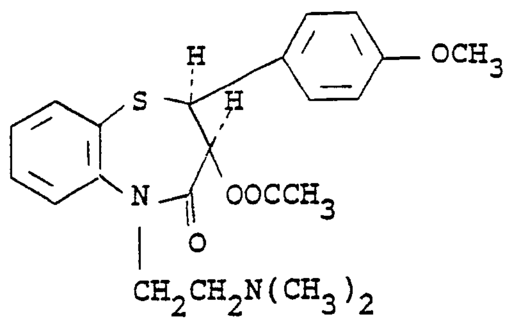

.HCl

4-Phenyl-1,4-dihydropyridine calcium antagonists may be employed which will have the structure

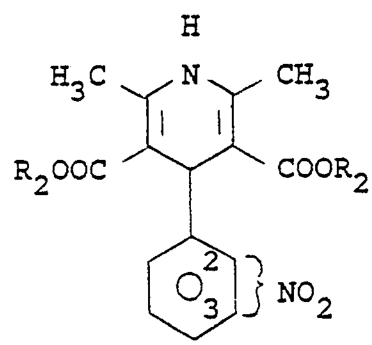

wherein $R_1$ and $R_2$ may be the same or different and are lower alkyl or lower alkoxy (lower alkyl) where lower alkyl and lower alkoxy contain 1 to 4 carbons.

The above compounds and methods for preparing same are disclosed in U. S. Patents Nos. 3,644,627, 3,485,847, 3,488,359, 3,574,843, 3,799,934, 3,932,645 and 4,154,839 which are incorporated herein by reference.

The dihydropyridine calcium antagonist present in the composition of the invention will preferably be nifedipine, that is, the compound of fromula C wherein $R_1$ is $CH_3$, $R_2$ is $CH_3$ and $NO_2$ is at the 2-position, namely,

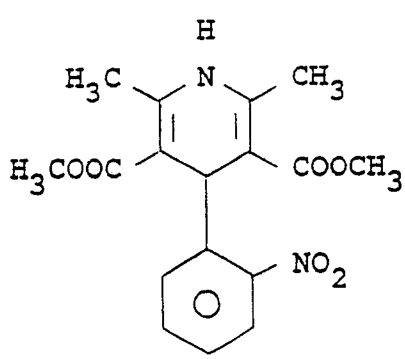

which is diclosed in U. S. Patents Nos. 3,644,627 and 3,485,847.

Other preferred 4-phenyl-1,4-dihydropyridine calcium antagonists suitable for use herein include niludipine, that is, the compound of formula C wherein $R_1$ is $-(CH_2)_2OC_3H_7$, $R_2$ is $-(CH_2)_2OC_3H_7$ and $NO_2$ is at the 3-position (disclosed in U. S. Patents Nos. 3,488,359 and 3,574,843); nimodipine, that is the compound of formula C wherein $R_1$ is $-(CH_2)_2OCH_3$, $R_2$ is $-CH(CH_3)_2$ and $NO_2$ is at the 3-position (disclosed in U.S. Patents Nos. 3,799,934 and 3,932,645); nitrendipine, that is, the compound of formula C wherein $R_1$ is $-CH_2CH_3$, $R_2$ is $-CH_3$ and $NO_2$ is at the 3-position (disclosed in U. S. Patents Nos. 3,799,934 and 3,932,645); and nisoldipine, that is, the compound of formula C wherein $R_1$ is $-CH_3$, $R_2$ is $-CH_2CH-(CH_3)_2$ and $NO_2$ is at the 2-position (disclosed in U. S. Patents Nos. 3,799,934, 3,932,645 and 4,154,839). Verapamil may also be employed.

The disclosure of the above-mentioned patents and references are incorporated herein by reference.

In carrying out the method of the present invention, the angiotensin converting enzyme inhibitor alone or in combination with the calcium channel blocker may be administered to mammalian species, such as monkeys, dogs, cats, rats and humans, and as such may be incorporated in a conventional systemic dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid of sodium bisulfite) or the like. Oral dosage forms are preferred, although parenteral forms such as intramuscular, intraperitoneal, or intravenous are quite satisfactory as well.

The dose administered must be carefully adjusted according to age, weight and condition of the patient, as well as the route of administration, dosage form and regimen and the desired result.

Thus, for oral administration, a satisfactory result may be obtained employing the ACE inhibitor in an amount within the range of from about 0.01 mg/kg to about 100 mg/kg and preferably from about 0.1 mg/kg to about 25 mg/kg alone or in combination with the calcium channel blocker in an amount within the range of from about 0.01 mg/kg to about 100 mg/kg and preferably from about 0.1 mg/kg to about 25 mg/kg with the ACE inhibitor and calcium channel blocker being employed together in the same oral dosage form or in separate oral dosage forms taken at the same time.

A preferred oral dosage form , such as tablets or capsules, will contain the ACE inhibitor in an amount of from about 0.1 to about 500 mg, preferably from about 125 to about 200 mg, and more preferably from about 25 to about 150 mg, alone or with the calcium channel blocker in an amount of from about 1 to about 350 mg, preferably from about 2 to about 200 mg, and more preferably from about 30 to about 150 mg.

For parenteral administration, the ACE inhibitor will be employed in an amount within the range of from about 0.005 mg/kg to about 10 mg/kg and preferably from about 0.01 mg/kg to about 1 mg/kg, alone or with the calcium channel blocker in an amount within the range of from about 0.005 mg/kg to about 20 mg/kg and preferably from about 0.01 mg/kg to about 2 mg/kg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 50 to 700 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonsful.

Such dosage forms can be administered to the patient on a regimen of one to four doses per day.

According to another modification, in order to more finely regulate the dosage schedule, the active substances may be administered separately in individual dosage units at the same time or carefully coordinated times. Since blood levels are built up and maintained by a regulated schedule of administration, the same result is achieved by the simultaneous presence of the two substances. The respective substances can be individually formulated in separate unit dosage forms in a manner similar to that described above.

Fixed combinations of ACE inhibitor and calcium channel blocker are more convenient and are preferred, especially in tablet or capsule form for oral administration.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, lactose or saccharin; a flavoring agent such as orange, peppermint, oil or wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Many of the active substances described above form commonly known, pharmaceutically acceptable salts such as alkali metal and other common basic salts or acid addition salts, etc. References to the base

substances are therefore intended to include those common salts known to be substantially equivalent to the parent compound.

The formulations as described above will be administered for a prolonged period, that is, for as long as the potential for onset of a migraine headache remains or the symptoms of a migraine headache continue. Sustained release forms of such formulations which may provide such amounts biweekly, weekly, monthly and the like may also be employed. A dosing period of at least two weeks and preferably at least 4 to 6 weeks are required to achieve minimal benefit.

The following Examples represent preferred embodiments of the present invention.

## Example 1

A captopril formulation suitable for oral administration in inhibiting onset of or treating a migraine headache is set out below.

1000 tablets each containing 25 mg of 1-[(2S)-3-mercapto-2-methylpropionyl]-L-proline were produced from the following ingredients.

| | |
|---|---|
| 1-[(2S)-3-Mercapto-2-methylpropionyl]-L-proline (captopril) | 25 g |
| Corn starch | 50 g |
| Gelatin | 7.5 g |
| Avicel (microcrystalline cellulose) | 25 g |
| Magnesium stearate | 2.5 g |

The captopril and corn starch are admixed with an aqueous solution of the gelatin. The mixture is dried and ground to a fine powder. The Avicel and then the magnesium stearate are admixed with the granulation. This is then compressed in a table to form 1000 tablets each containing 25 mg of active ingredient which is used for inhibiting onset of or treating migraine headache.

## Example 2

By substituting 50 g of 1-(3-mercapto-2-D-methylpropanoyl)-L-proline for the captopril in Example 1, 1000 tablets each containing 50 mg of the 1-(3-mercapto-2-D-methylpropanoyl)-L_proline are produced which is useful in treating or inhibiting onset of migraine headache.

## Example 3

100 tablets each containing 100 mg of captopril and 500 mg carbamazepine are produced from the following ingredients:

| | |
|---|---|
| Captopril | 100 g |
| Lactose | 100 g |
| Avicel | 150 g |
| Corn starch | 50 g |
| Magnesium stearate | 5 g |

The captopril, lactose and Avicel are admixed, then blended with the corn starch. Magnesium stearate is added. The dry mixture is compressed in a tablet press to form 1000 1205 mg tablets each containing

600 mg of active ingredients. The tablets are coated with a solution of Methocel E 15 (methyl cellulose) including as a color a lake containing yellow #6. The resulting tablets are useful in treating or inhibiting onset of migraine headache.

## Example 4

Two piece #1 gelatin capsules each containing 25 mg of captopril and 100 mg of carbamazepine are filled with a mixture of the following ingredients:

| | |
|---|---|
| Captopril | 25 mg |
| Magnesium stearate | 7 mg |
| USP lactose | 193 mg. |

The resulting capsules are useful in treating or inhibiting onset of migraine headache.

## Example 5

An injectable solution for use in treating or inhibiting onset of migraine headache trigeminal neuralgia is produced as follows:

| | |
|---|---|
| Captopril | 500 mg |
| Methyl paraben | 5 mg |
| Propyl paraben | 1 mg |
| Sodium chloride | 25 g |
| Water for injection qs. | 5 l. |

The captopril, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection for treating or inhibiting onset of migraine headache.

## Example 6

Tablets for use in treating or inhibiting onset of migraine headache are prepared as described in Example 1 except that N-(1-ethoxycarbonyl-3-phenylpropyl)-L-alanyl-L-proline (enalapril) is used in place of captopril.

## Example 7

An injectable for use in treating or inhibiting onset of migraine headache is prepared as described in Example 5 except that N-(1-ethoxycarbonyl-3-phenylpropyl)-L-alanyl-L-proline (enalapril) is employed in place of captopril.

## Example 8

A zofenopril formulation suitable for oral administration in treating or inhibiting onset of migraine headache is set out below.

1000 tables each containing 100 mg of zofenopril are produced from the following ingredients.

| | |
|---|---|
| [1(S),4(S)]-1-[3-(benzoylthio)-2-methyl-1-oxopropyl-4-(phenylthio)-L-proline (zofenopril) | 100 g |
| Corn starch | 50 g |
| Gelatin | 7.5 g |
| Avicel (microcrystalline cellulose) | 25 g |
| Magnesium stearate | 2.5 g |

The zofenopril and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 100 mg of active ingredient which is used for treating or inhibiting onset of migraine headache.

## Example 9

By substituting 100 g of fosinopril for the zofenopril in Example 8, 1000 tablets each containing 100 mg of the fosinopril are produced which is useful in treating or inhibiting onset of migraine headache.

## Example 10

1000 tablets each containing 200 mg of fosinopril and 200 mg of carbamazepine are produced from the following ingredients:

| | |
|---|---|
| Fosinopril | 100 g |
| Lactose | 100 g |
| Avicel | 150 g |
| Corn starch | 50 g |
| Magnesium stearate | 5 g |

The fosinopril, carbamozepine, lactose and Avicel are admixed, then blended with the corn starch. Magnesium stearate is added. The dry mixture is compressed in a table press to form 1000 705 mg tablets each containing 300 mg of active ingredients. The tablets are coated with a solution of Methocel E 15 (methyl cellulose) including as a color a lake containing yellow #6. The resulting tablets are useful in treating or inhibiting onset of migraine headache.

## Example 11

Tablets for use in treating or inhibiting onset of migraine headache are prepared as described in Example 1 except that 1-[N[hydroxy-(4-phenylbutyl)phosphinyl]-L-alanyl-L-proline, disodium salt (prepared as described in U. S. Patent No. 4,432,971) is used in place of captopril.

## Example 12

An injectable for use in treating or inhibiting onset of migraine headache is prepared as described in Example 5 except that 1-[N-[hydroxy(4-phenylbutyl)phosphinyl]-L-alanyl]-L-proline, disodium salt (prepared as described in U. S. Patent No. 4,432,971) is used in place of captopril.

Example 13

A captopril-diltiazem formulation suitable for oral administration in the treatment of migraine headache is set out below.

1000 tablets each containing 100 mg of 1-[(2S)-3-mercapto-2-methylpropionyl]-L-proline and 100 mg of diltiazem are produced from the following ingredients:

| | |
|---|---|
| 1-(2S)-3-mercapto-2-methylpropionyl]-L-proline (captopril) | 100 g |
| Diltiazem | 100 g |
| Corn Starch | 50 g |
| Gelatin | 7.5 g |
| Avicel (microcrystalline cellulose) | 25 g |
| Magnesium stearate | 2.5 g |

· The captopril, diltiazem and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 200 mg of active ingredients which is used for preventing or treating migraine headache.

Example 14

By substituting 100 g of 1-(3-mercapto-2-D-methylpropanoyl)-L-proline for the captopril in Example 13, 1000 tablets each containing 100 mg of the 1-(3-mercapto-2-D-methylpropanoyl)-L-proline and 100 mg diltiazem are produced which is useful in preventing or treating migraine headache.

Example 15

1000 tablets each containing 200 mg of captopril and 200 mg nifedipine are produced from the following ingredients:

| | |
|---|---|
| Captopril | 200 g |
| Nifedipine | 200 g |
| Lactose | 100 g |
| Avicel | 150 g |
| Corn starch | 50 g |
| Magnesium stearate | 5 g |

The captopril, diltiazem, lactose and Avicel are admixed, then blended with the corn starch. Magnesium stearate is added. The dry mixture is compressed in a tablet press to form 1000 505 mg tablets each containing 200 mg of each active ingredient. The tablets are coated with a solution of Methocel E 15 (methyl cellulose) including as a color a lake containing yellow #6. The resulting tablets are useful in preventing or treating migraine headache.

Example 16

Two piece #1 gelatin capsules each containing 250 mg of enalapril and 150 mg of nitrendipine are filled with a mixture of the following ingredients:

| | |
|---|---|
| Enalapril | 250 mg |
| Nitrendipine | 150 mg |
| Magnesium stearate | 7 mg |
| USP lactose | 193 mg |

The resulting capsules are useful in preventing or treating migraine headache.

Example 17

An injectable solution for use in treating or preventing migraine headache is produced as follows:

| | |
|---|---|
| Captopril | 500 mg |
| Diltiazem | 300 mg |
| Methyl paraben | 5 g |
| Propyl paraben | 1 g |
| Sodium chloride | 25 g |
| Water for injection qs. | 5 L. |

The captopril, diltiazem, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

Example 18

Tablets for use in preventing or treating migraine headache are prepared as described in Example 13 except that N-(1-ethoxycarbonyl-3-phenylpropyl)-L-alanyl-L-proline (enalapril) is used in place of catopril and nifedipine is used in place of diltiazem.

Example 19

Tablets for use in treating or preventing migrane headache are prepared following the procedure of Example 13 except that zofenopril is employed in place of captopril and nisoldipine is used in place of diltiazem.

Example 20

Tablets for use in treating or preventing migraine headache are prepared following the procedure of Example 13 except that fosinopril is employed in place of captopril.

Example 21

Tablets for use in treating or preventing migraine headache are prepared following the procedure of Example 13 except that alacepril is employed in place of captopril.

Example 22

Tablets for use in treating or preventing migraine headache are prepared following the procedure of Example 13 except that (S)-1-[6-amino-2-[[hydroxy-(4-phenylbutyl)phosphinyl]oxy]-1-oxohexyl]-L-proline or lisinopril is employed in place of captopril.

## Claims

1. Use of an angiotensin converting enzyme inhibitor in combination with a calcium channel blocker for preparing a medicament for inhibiting onset or treating migraine headache in a mammalian specie.

2. The use as defined in claim 1 wherein the angiotensin converting enzyme inhibitor is a phosphonate substituted amino or imino acid or salt thereof, a substituted proline derivative, a carboxyalkyl dipeptide derivative, a phosphinylalkanoyl proline derivative or a phosphonamidate derivative.

3. The use as defined in claim 1 wherein said medicament is suitable for oral or parenteral administration.

4. The use as defined in claim 1 wherein said angiotensin converting enzyme inhibitor in combination with a calcium channel blocker is admixed with a pharmaceutically acceptable carrier thereof.

5. The use as defined in claim 1 wherein said angiotensin converting enzyme inhibitor is a substituted proline derivative.

6. The use as defined in claim 1 wherein said angiotensin converting enzyme inhibitor is a carboxyalkyl dipeptide derivative.

7. The use as defined in claim 1 wherein said angiotensin converting enzyme inhibitor is a phosphinylalkanoyl proline derivative, a phosphoramidate derivative, or a phosphonate substituted amino or imino acid or salt thereof.

8. The use as defined in claim 1 wherein said medicament is in a dosage form wherein said angiotensin converting enzyme inhibitor is captopril, in combination with a calcium channel blocker, in single or separate dosage forms.

9. The use as defined in claim 1 wherein said angiotensin converting enzyme inhibitor is enalapril.

10. The use as defined in claim 1 wherein said angiotensin converting enzyme inhibitor is 1- [N-[hydroxy(4-phenylbutyl)-phosphinyl] -2-alanyl]-L-proline or its disodium salt.

11. The use as defined in claim 1 wherein said medicament comprises dosage forms including said angiotensin converting enzyme inhibitor in the amount of from about 0.1 to about 500 mg and the calcium channel blocker is in the same or separate dosage forms in the amount of from about 1 to about 300 mg.

12. The use as defined in claim 1 wherein said angiotensin converting enzyme inhibitor is captopril and said medicament consists of dosage forms for sistemical administration comprising an amount of from about 0.1 to about 500 mg of captopril.

13. The use as defined in claim 1 wherein said medicament comprises the angiotensin converting enzyme inhibitor together with a calcium channel blocker.

14. The use as defined in claim 13, wherein the calcium channel blocker is diltiazem or a 4-phenyl-1,4-dihydropyridine.

15. The use as defined in claim 14 wherein the 4-phenyl-1,4-dihydropyridine is nifedipine.

16. The use as defined in claim 13 wherein said medicament includes the angiotensin converting enzyme inhibitor employed in a weight ratio to the calcium channel blocker of within the range of from about 0.1:1 to about 10:1.

17. A pharmaceutical produce containing an angiotensin converting enzyme inhibitor and a calcium channel blocker as a combined preparation for simultaneous, separate or sequential use for inhibiting onset of or treating migraine headache in a mammalian specie.